# EUROPEAN PATENT APPLICATION

(11) **EP 2 977 115 A1**
(43) Date of publication of application: **27.01.2016**
(21) Application number: 14769702.3
(22) Date of filing: 21.02.2014
(51) Int. Cl.: B06B 1/06

(54) **LAMINATED ULTRASONIC VIBRATION DEVICE, METHOD FOR MANUFACTURING LAMINATED ULTRASONIC VIBRATION DEVICE, AND ULTRASONIC MEDICAL APPARATUS**

(30) Priority: 18.03.2013 JP 2013055504
(71) Applicant: Olympus Corporation, Shibuya-ku Tokyo 151-0072 (JP)
(72) Inventor: ITO Hiroshi, Tokyo 151-0072 (JP)
(74) Representative: Winter, Brandl, Fürniss, Hübner, Röss, Kaiser, Polte - Partnerschaft mbB
(86) International application number: PCT/JP2014/054135
(87) International publication number: WO 2014/148196

(57) **Abstract**

A multilayer ultrasound vibration device 2 includes a plurality of piezoelectric bodies and a plurality of electrode layers stacked together, wherein: the plurality of piezoelectric bodies are formed of a plurality of monocrystalline piezoelectric bodies 61; and widths d2 and d3 of outer surfaces 62a and 63a of the plurality of electrode layers 62 and 63 in a stacking direction is made larger than a spacing distance d1 of the stacked plurality of monocrystalline piezoelectric bodies 61 in the stacking direction.

## Description

### Technical Field

The present invention relates to a multilayer ultrasound vibration device adapted to excite ultrasound vibrations, a production method for the multilayer ultrasound vibration device and an ultrasound medical apparatus equipped with the multilayer ultrasound vibration device.

### Background Art

Examples of ultrasound treatment instruments which perform coagulation/dissection treatment of living tissue using ultrasound vibrations include one which incorporates a bolt-clamped Langevin transducer as an ultrasound vibration source in a handpiece.

Such a bolt-clamped Langevin transducer is disclosed, for example, in Japanese Patent Application Laid-Open Publication No. 2009-220014, in which a piezoelectric element adapted to convert an electrical signal into mechanical vibrations is sandwiched between a front mass and back mass of metal members, fastened securely by bolts, and thereby integrated to vibrate as an integral whole.

Now, a transducer in which a piezoelectric element and metal members vibrate integrally with the piezoelectric element by being sandwiched between metal members, and integrated with metal members by adhesive or some other means is known as a Langevin transducer. A transducer for which bolting is used as a method for integrating the piezoelectric element with the metal members is known as a bolt-clamped Langevin transducer. Regarding a typical configuration of a conventional bolt-clamped Langevin transducer, lead zirconate titanate (PZT, Pb(Zrx,Ti₁₋ₓ)O3) is used for the piezoelectric device, which is shaped as a ring, through which a bolt is passed.

PZT has high productivity and high electromechanical conversion efficiency as well as excellent properties as a piezoelectric material, and thus has been used in various fields including those of the ultrasound transducer and actuator for many years. However, since PZT uses lead, due to concerns over adverse environmental effects, recently there has been demand for non-lead piezoelectric material which does not use lead.

One of non-lead piezoelectric materials having high electromechanical conversion efficiency suitable for ultrasound transducers for high-output applications used in ultrasound treatment instruments is monocrystalline piezoelectric LiNb03 (lithium niobate monocrystal). LiNb03 does not have good machinability, and thus requires inexpensive machining and assembly methods when used for a Langevin transducer or bolt-clamped Langevin transducer.

Methods for inexpensively creating a Langevin transducer using LiNb03 or another piezoelectric monocrystal include a method configured to bond individual members (monocrystalline piezoelectric element, insulating plate, and metal members) into a single piece using a metal material as a bonding material through a molten state.

Now, to drive a Langevin transducer which uses a piezoelectric monocrystal, it is necessary to apply a drive signal to each of stacked monocrystalline piezoelectric elements. In the case of a bonded Langevin transducer in which a metal material is used among individual ones of the stacked monocrystalline piezoelectric elements as a bonding material, when a wiring line is connected to the bonding material, a drive signal can be applied to each of the monocrystalline piezoelectric elements.

However, there is a problem in that the bonding material made of metal material has a small thickness, resulting in a small area of an electrical connection portion between the bonding material and wiring line and thereby tending to reduce reliability of electrical connection. In contrast, preferably the bonding material has a small thickness from a viewpoint of vibration performance, and so there is a trade-off relation between the reliability of electrical connection and ultrasound vibration performance.

Thus, the present invention has been made in view of the above circumstances and has an object to provide a multilayer ultrasound vibration device which improves reliability of electrical connection in that part which forms the electrical connection between a wiring line adapted to apply a drive signal of a Langevin transducer and metallic bonding material while preventing degradation of vibration performance as well as to provide a production method for the multilayer ultrasound vibration device and an ultrasound medical apparatus equipped with the multilayer ultrasound vibration device.

### Disclosure of Invention

### Means for Solving the Problem

According to one aspect of the present invention, there is provided a multilayer ultrasound vibration device, comprising a plurality of piezoelectric bodies and a plurality of electrode layers stacked together, wherein: the plurality of piezoelectric bodies are formed of a plurality of monocrystalline piezoelectric bodies; and width of outer surfaces of the plurality of electrode layers in a stacking direction is made larger than a spacing distance of the stacked plurality of monocrystalline piezoelectric bodies in the stacking direction.

Also, according to one aspect of the present invention, there is provided a production method for a multilayer ultrasound vibration device which comprises a plurality of piezoelectric bodies and a plurality of electrode layers stacked together, in which: the plurality of piezoelectric bodies are formed of a plurality of monocrystalline piezoelectric bodies; and width of outer surfaces of the plurality of electrode layers in a stacking direction is made larger than a spacing distance of the stacked plurality of monocrystalline piezoelectric bodies in the stacking direction, the production method comprising: forming V-shaped grooved portions in front and back surfaces of a monocrystalline piezoelectric wafer by a dicing process; dicing the monocrystalline piezoelectric wafer into the plurality of monocrystalline piezoelectric bodies along centers of the grooved portions by the dicing process while making the chamfered portions out of the cut grooved portions; and placing a bonding metal on the plurality of monocrystalline piezoelectric bodies, applying a heating and cooling process thereto, thereby bonding the plurality of monocrystalline piezoelectric bodies into one body, and forming the plurality of electrode layers from the bonding metal.

Furthermore, according to one aspect of the present invention, there is provided an ultrasound medical apparatus, comprising a multilayer ultrasound vibration device; and a probe distal end portion, wherein in the multilayer ultrasound vibration device, a plurality of piezoelectric bodies and a plurality of electrode layers are stacked together, the plurality of piezoelectric bodies are formed of a plurality of monocrystalline piezoelectric bodies, and width of outer surfaces of the plurality of electrode layers in a stacking direction is made larger than a spacing distance of the stacked plurality of monocrystalline piezoelectric bodies in the stacking direction; and the probe distal end portion is used to treat living tissue with ultrasound vibrations generated by the multilayer ultrasound vibration device and transmitted to the probe distal end portion.

The present invention described above can provide a multilayer ultrasound vibration device which improves reliability of electrical connection in that part which forms the electrical connection between a wiring line adapted to apply a drive signal of a Langevin transducer and metallic bonding material while preventing degradation of vibration performance as well as to provide a production method for the multilayer ultrasound vibration device and an ultrasound medical apparatus equipped with the multilayer ultrasound vibration device.

### Brief Description of the Drawings

Fig. 1 is a sectional view showing an overall configuration of an ultrasound medical apparatus according to a first embodiment of the present invention;
Fig. 2 is a diagram showing an overall schematic configuration of a transducer unit according to the first embodiment of the present invention;
Fig. 3 is a sectional view showing an overall configuration of an ultrasound medical apparatus according to another aspect of the first embodiment of the present invention;
Fig. 4 is a perspective view showing the configuration of the ultrasound transducer according to the first embodiment of the present invention;
Fig. 5 is a side view showing the configuration of the ultrasound transducer according to the first embodiment of the present invention;
Fig. 6 is a plan view showing a configuration of a LiNb03 wafer according to the first embodiment of the present invention;
Fig. 7 is a side view showing the configuration of the LiNb03 wafer according to the first embodiment of the present invention;
Fig. 8 is a side view showing the configuration of the LiNb03 wafer with grooves formed therein according to the first embodiment of the present invention;
Fig. 9 is a sectional view showing the configuration of the LiNb03 wafer with a film of underlying metal formed thereon according to the first embodiment of the present invention;
Fig. 10 is a sectional view showing a configuration of plural monocrystalline piezoelectric chips cut from the LiNb03 wafer according to the first embodiment of the present invention;
Fig. 11 is a perspective view showing the configuration of the monocrystalline piezoelectric chip according to the first embodiment of the present invention;
Fig. 12 is an exploded perspective view showing the configuration of the ultrasound transducer according to the first embodiment of the present invention;
Fig. 13 is a perspective view showing the configuration of the ultrasound transducer according to the first embodiment of the present invention;
Fig. 14 is a sectional view of the ultrasound transducer for describing thicknesses of positive-side bonding metal and negative-side bonding metal according to the first embodiment of the present invention;
Fig. 15 is a sectional view showing the configuration of the ultrasound transducer according to the first embodiment of the present invention;
Fig. 16 is a perspective view showing the configuration of the ultrasound transducer according to the first embodiment of the present invention;
Fig. 17 is an exploded perspective view showing a configuration of an ultrasound transducer according to a modification of the first embodiment of the present invention;
Fig. 18 is a plan view showing a surface of a LiNb03 wafer according to a second embodiment of the present invention;
Fig. 19 is a plan view showing a back surface of the LiNb03 wafer according to the second embodiment of the present invention;
Fig. 20 is a side view showing a configuration of the LiNb03 wafer according to the second embodiment of the present invention;
Fig. 21 is a side view showing the configuration of the LiNb03 wafer with grooves formed therein according to the second embodiment of the present invention;
Fig. 22 is a sectional view showing the configuration of the LiNb03 wafer with a film of underlying metal formed thereon according to the second embodiment of the present invention;
Fig. 23 is a sectional view showing a configuration of plural monocrystalline piezoelectric chips cut from the LiNb03 wafer according to the second embodiment of the present invention;
Fig. 24 is a perspective view showing the configuration of the monocrystalline piezoelectric chip according to the second embodiment of the present invention;
Fig. 25 is an exploded perspective view showing a configuration of four monocrystalline piezoelectric chips according to the second embodiment of the present invention;
Fig. 26 is a sectional view showing the configuration of the ultrasound transducer according to the second embodiment of the present invention;
Fig. 27 is a perspective view showing the configuration of the ultrasound transducer according to the second embodiment of the present invention;
Fig. 28 is a perspective view showing a configuration of an ultrasound transducer according to a first modification of the second embodiment of the present invention; and
Fig. 29 is a perspective view showing a configuration of an ultrasound transducer according to a second modification of the second embodiment of the present invention.

### Best Mode for Carrying Out the Invention

The present invention will be described below with reference to the drawings. In the following description, it should be noted that the drawings in each embodiment are schematic, that a relationship between thickness and width of each component as well as ratios of the thickness among individual components are different from actual ones, and that dimensional relationships or ratios may not be uniform among the drawings.

### (First Embodiment)

To begin with, a first embodiment of the present invention will be described below with reference to drawings.

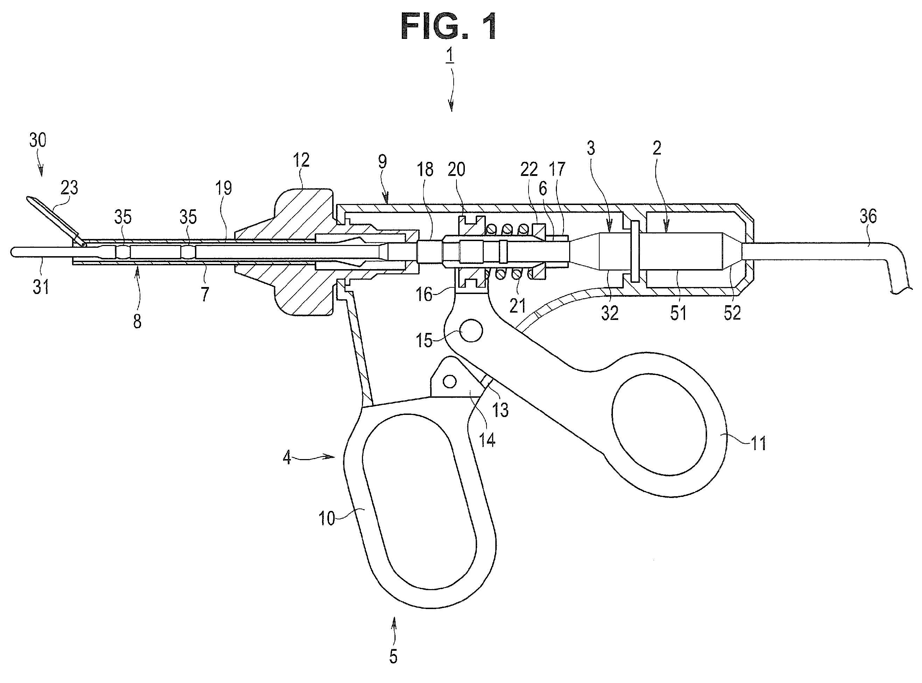

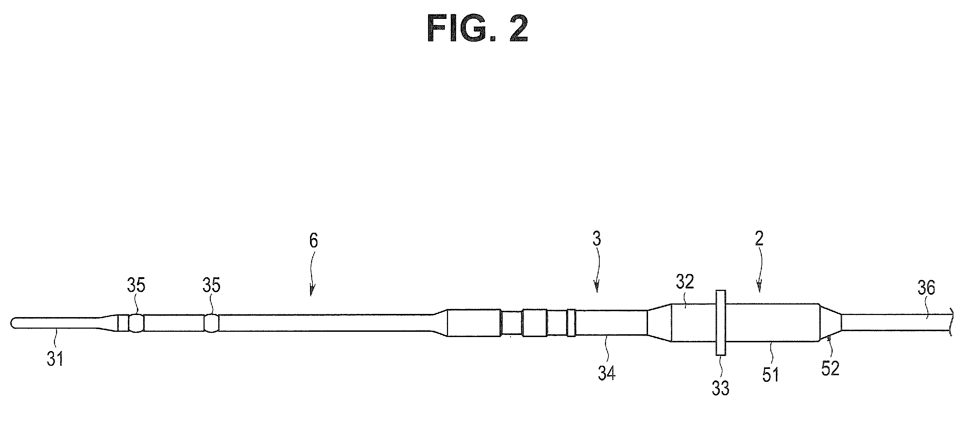

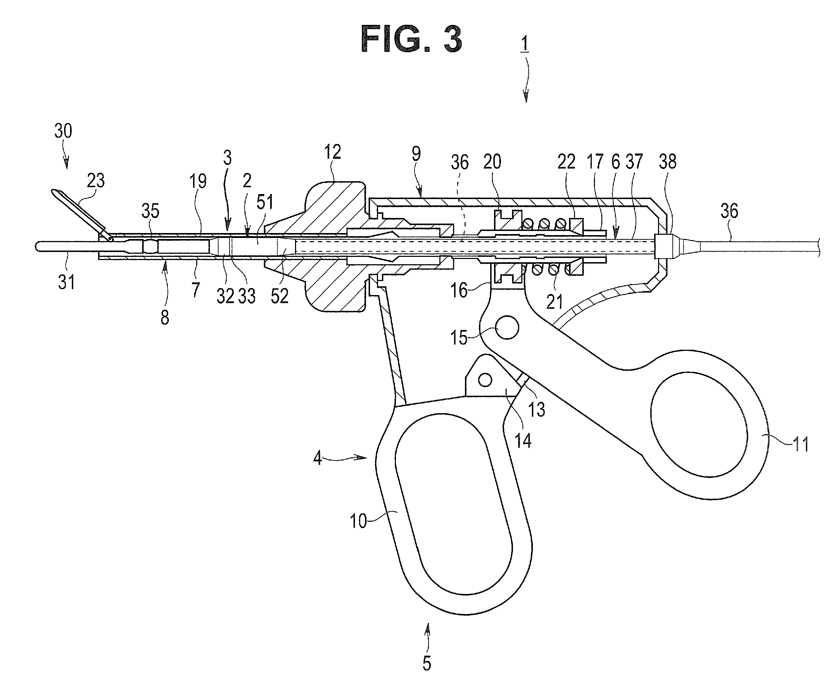

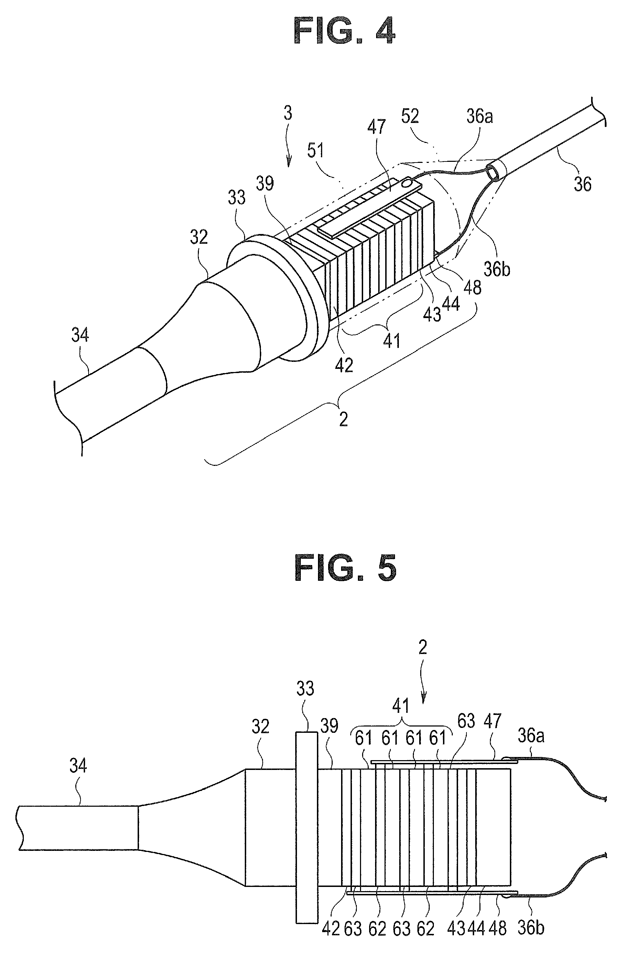

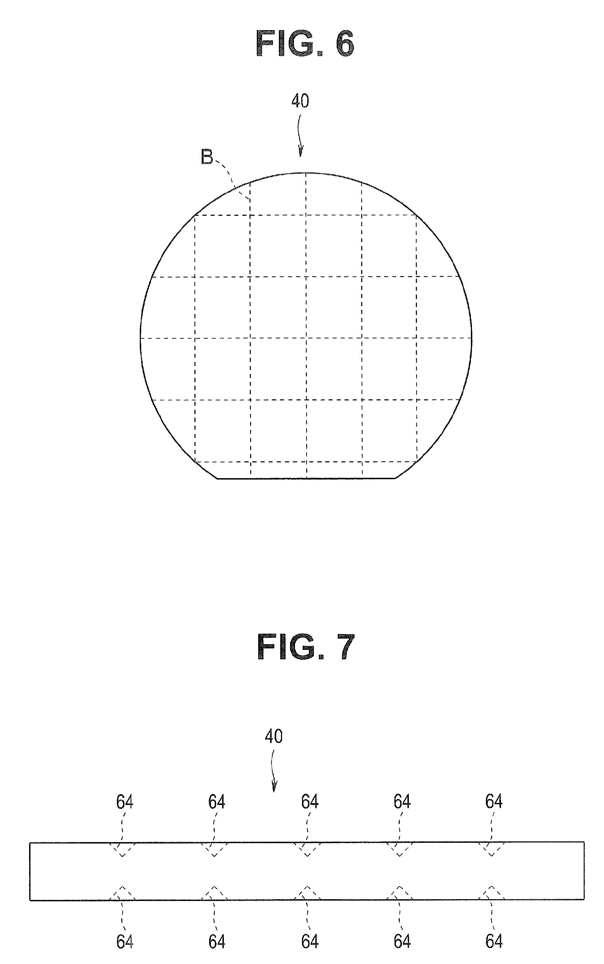

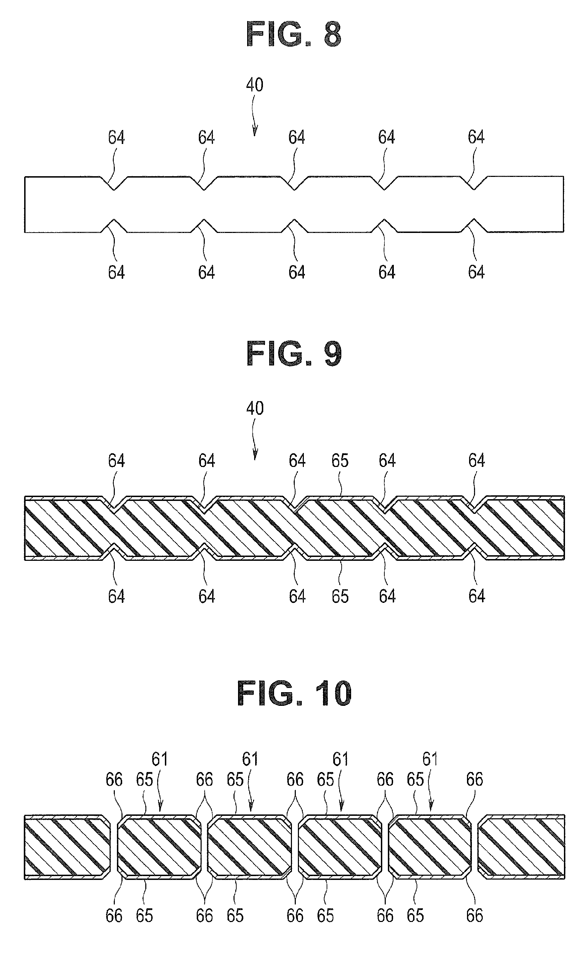

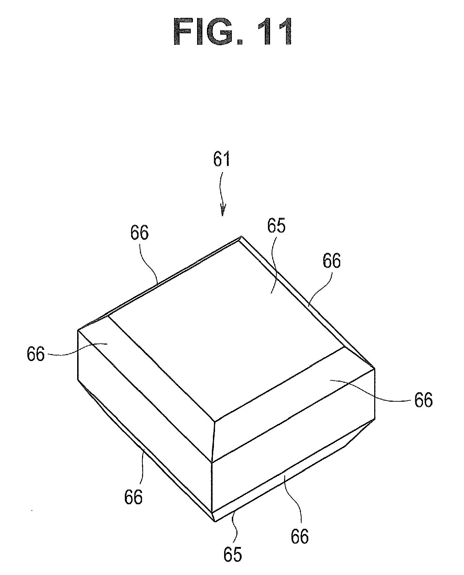

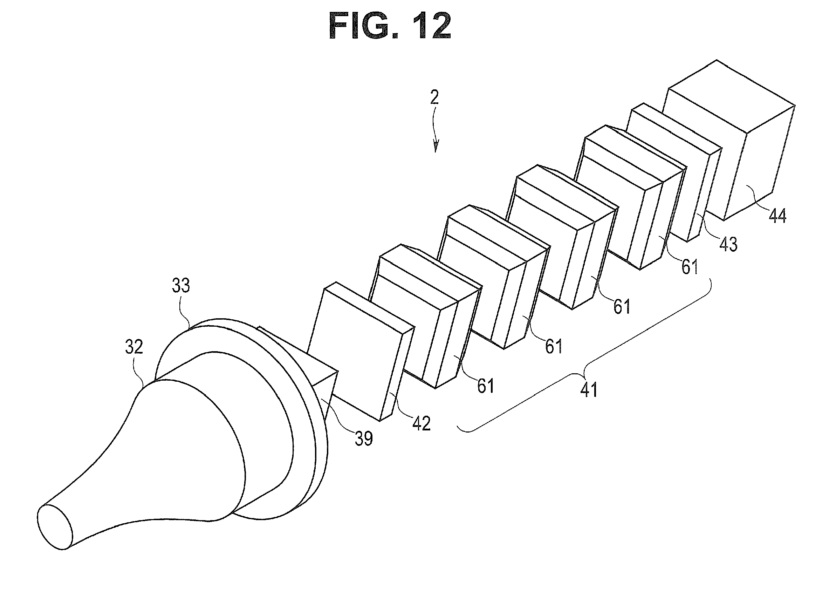

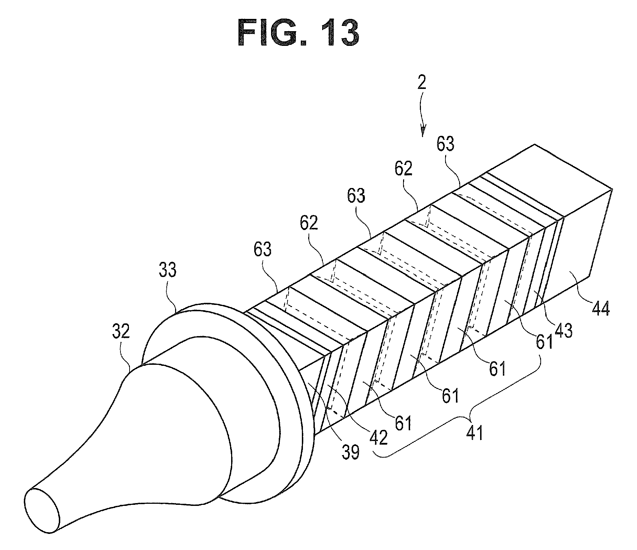

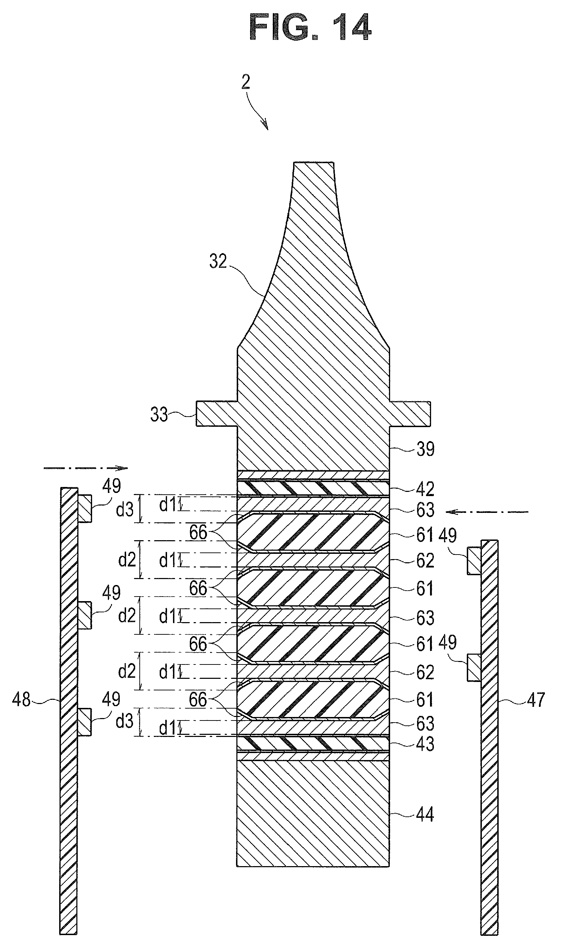

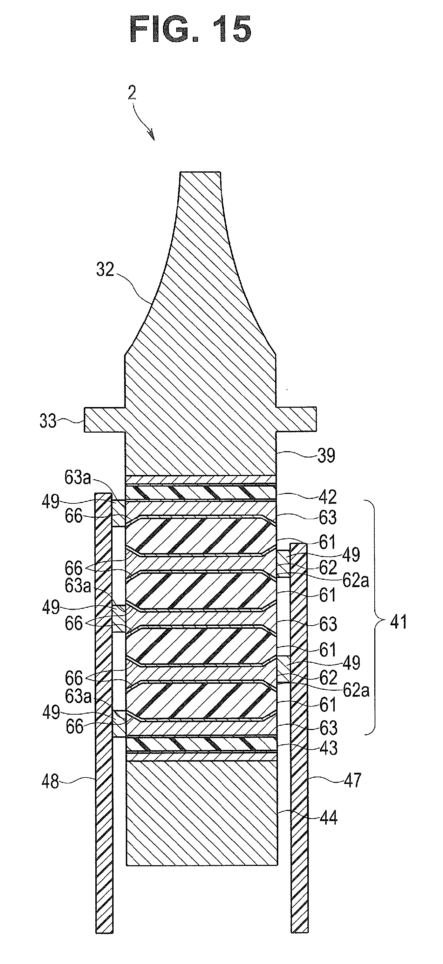

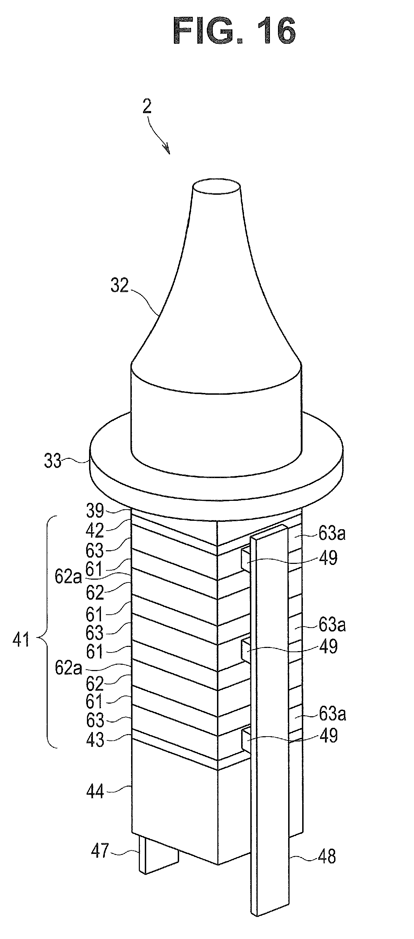

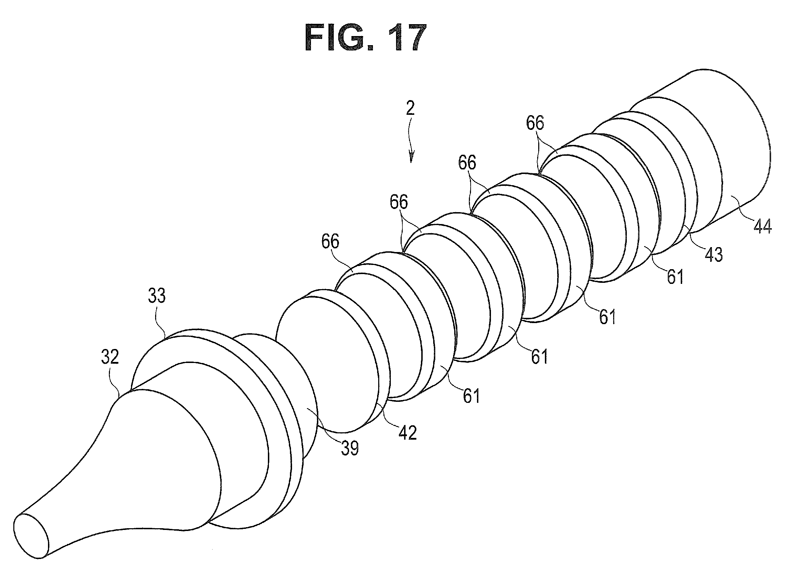

Fig. 1 is a sectional view showing an overall configuration of an ultrasound medical apparatus, Fig. 2 is a diagram showing an overall schematic configuration of a transducer unit, Fig. 3 is a sectional view showing an overall configuration of an ultrasound medical apparatus according to another aspect, Fig. 4 is a perspective view showing the configuration of the ultrasound transducer, Fig. 5 is a side view showing the configuration of the ultrasound transducer, Fig. 6 is a plan view showing a configuration of a LiNbO3 wafer, Fig. 7 is a side view showing the configuration of the LiNb03 wafer, Fig. 8 is a side view showing the configuration of the LiNb03 wafer with grooves formed therein, Fig. 9 is a sectional view showing the configuration of the LiNb03 wafer with a film of underlying metal formed thereon, Fig. 10 is a sectional view showing a configuration of plural monocrystalline piezoelectric chips cut from the LiNb03 wafer, Fig. 11 is a perspective view showing the configuration of the monocrystalline piezoelectric chip, Fig. 12 is an exploded perspective view showing the configuration of the ultrasound transducer, Fig. 13 is a perspective view showing the configuration of the ultrasound transducer, Fig. 14 is a sectional view of the ultrasound transducer for describing thicknesses of positive-side bonding metal and negative-side bonding metal, Fig. 15 is a sectional view showing the configuration of the ultrasound transducer, Fig. 16 is a perspective view showing the configuration of the ultrasound transducer, and Fig. 17 is an exploded perspective view showing a configuration of an ultrasound transducer according to a modification.

### (Ultrasound Medical Apparatus)

An ultrasound medical apparatus 1 shown in Fig. 1 mainly includes a transducer unit 3 provided with an ultrasound transducer 2 adapted to generate ultrasound vibrations, and a handle unit 4 used to treat an affected part using the ultrasound vibrations.

The handle unit 4 includes an operation portion 5, an insertion sheath portion 8, and a distal end treatment portion 30, where the insertion sheath portion 8 is made up of a long cannula 7. A proximal end portion of the insertion sheath portion 8 is mounted axially rotatably on the operation portion 5. The distal end treatment portion 30 is provided at a distal end of the insertion sheath portion 8. The operation portion 5 of the handle unit 4 includes an operation portion body 9, a fixed handle 10, a movable handle 11, and a rotation knob 12. The operation portion body 9 is formed integrally with the fixed handle 10.

A slit 13 through which the movable handle 11 is passed is formed in a rear side of a coupling portion between the operation portion body 9 and fixed handle 10. An upper part of the movable handle 11 extends into the operation portion body 9 through the slit 13. A handle stopper 14 is fixed to a lower-side end portion of the slit 13. The movable handle 11 is pivotally mounted on the operation portion body 9 via a handle support shaft 15. Along with an operation in which the movable handle 11 pivots around the handle support shaft 15, the movable handle 11 is designed to be opened and closed with respect to the fixed handle 10.

A substantially U-shaped coupling arm 16 is provided in an upper end portion of the movable handle 11. Also, the insertion sheath portion 8 includes the cannula 7 and an operation pipe 17 passed through the cannula 7 so as to be movable in an axial direction. A large-diameter portion 18 larger in diameter than a distal end side portion is provided in a proximal end portion of the cannula 7. The rotation knob 12 is designed to be mounted around the large-diameter portion 18.

A ring-shaped slider 20 is provided on an outer circumferential face of the operation pipe 19 in such a way as to be movable along the axial direction. A fixing ring 22 is disposed behind the slider 20 via a coil spring (elastic member) 21.

Furthermore, a proximal end portion of a grasping portion 23 is pivotally coupled to a distal end portion of the operation pipe 19 via an action pin. In conjunction with a distal end portion 31 of the probe 6, the grasping portion 23 makes up a treatment portion of the ultrasound medical apparatus 1. During an operation in which the operation pipe 19 moves in the axial direction, the grasping portion 23 is operated so as to be pushed and pulled in a forward and backward direction via the action pin. In so doing, in an operation in which the operation pipe 19 is operated so as to move toward a hand side, the grasping portion 23 is pivoted in a counterclockwise direction around a fulcrum pin via the action pin. Consequently, the grasping portion 23 pivots in a direction toward the distal end portion 31 of the probe 6 (in a closing direction). At this time, living tissue can be grasped between the grasping portion 23 of a single swing type and the distal end portion 31 of the probe 6.

With the living tissue grasped in this way, the ultrasound transducer 2 is caused to vibrate by supplying electric power from an ultrasound power source to the ultrasound transducer 2. The ultrasound vibrations are transmitted to the distal end portion 31 of the probe 6. Then, the living tissue being grasped between the grasping portion 23 and the distal end portion 31 of the probe 6 is treated using the ultrasound vibrations.

### (Transducer Unit)

Now, the transducer unit 3 will be described.

As shown in Fig. 2, the transducer unit 3 is an integral assembly of the ultrasound transducer 2 and the probe 6 which is a rod-shaped vibration transmission member adapted to transmit the ultrasound vibrations generated by the ultrasound transducer 2.

A horn 32 adapted to amplify amplitude of the ultrasound transducer is provided consecutively with the ultrasound transducer 2. The horn 32 is formed of duralumin or a titanium alloy such as 64Ti (Ti-6Al-4V). The horn 32 is formed into a conical shape with an outside diameter being reduced toward a distal end side and an outward flange 33 is formed in an outer circumferential portion of a proximal end. Note that shape of the horn 32 is not limited to a conical shape and may be an exponential shape with the outside diameter being reduced exponentially toward the distal end side or a stepped shape with the outside diameter being reduced stepwise toward the distal end side.

The probe 6 includes a probe body 34 formed, for example, of a titanium alloy such as 64Ti (Ti-6Al-4V). The ultrasound transducer 2 provided consecutively with the above-mentioned horn 32 is disposed on the side of a proximal end portion of the probe body 34. In this way, the transducer unit 3 is formed by integrating the probe 6 and ultrasound transducer 2. Note that the probe 6 includes the probe body 34 and horn 32, which are bonded by being screwed together.

The ultrasound vibrations generated by the ultrasound transducer 2 are designed to be amplified by the horn 32 and then transmitted to the side of the distal end portion 31 of the probe 6. A treatment portion used to treat living tissue and described later is formed in the distal end portion 31 of the probe 6.

Also, two rubber linings 35 formed of an elastic member into ring shapes are mounted at an interval on an outer circumferential face of the probe body 34 at a few locations corresponding to vibration node positions halfway in the axial direction. The rubber linings 35 are designed to prevent contact between the outer circumferential face of the probe body 34 and the operation pipe 19 described later. That is, the probe 6, which is a transducer-integrated probe, is inserted into the operation pipe 19 during assembly of the insertion sheath portion 8. By so doing, the rubber linings 35 prevent contact between the outer circumferential face of the probe body 34 and operation pipe 19.

Also, the ultrasound transducer 2 is electrically connected to an unillustrated power supply main body via an electric cable 36, where the power supply main body is adapted to supply electric current to generate ultrasound vibrations. The ultrasound transducer 2 is driven when electric power is supplied from the power supply main body to the ultrasound transducer 2 through wiring lines in the electric cable 36. Note that the transducer unit 3 includes the ultrasound transducer 2 adapted to generate ultrasound vibrations, the horn 32 adapted to amplify the generated ultrasound vibrations, and the probe 6 adapted to transmit the amplified ultrasound vibrations.

Note that the ultrasound transducer 2 and transducer unit 3 do not necessarily have to be housed in the operation portion body 9 as shown in Fig. 1, and may be housed, for example, in the operation pipe 19 as shown in Fig. 3. In the ultrasound medical apparatus 1 of Fig. 3, the electric cable 36 running from a bend preventer 52 of the ultrasound transducer 2 to a connector 38 disposed in a base portion of the operation portion body 9 is housed by being passed through a metal pipe 37. Here, the connector 38 is not indispensable, and the electric cable 36 may be configured to be extended into the operation portion body 9 and connected to the bend preventer 52 of the ultrasound transducer 2 directly. With a configuration such as shown in Fig. 3, the ultrasound medical apparatus 1 can offer improved space-savings in the operation portion body 9. Note that functions of the ultrasound medical apparatus 1 in Fig. 3 are similar to those in Fig. 1, and thus detailed description thereof will be omitted.

### (Ultrasound Transducer)

Now, the ultrasound transducer 2 as a multilayer ultrasound vibration device according to the present invention will be described below.

As shown in Figs. 4 and 5, the ultrasound transducer 2 of the transducer unit 3 includes the horn 32, a multilayer transducer 41, and a cover body 51, starting from a distal end, where the horn 32 is connected to the probe body 34, which is one of vibration transmission members, by screwing or the like, the multilayer transducer 41 is rectangular in shape (quadrangular prism-shaped) here and provided consecutively behind the horn 32, and the cover body 51 covers the multilayer transducer 41 from a proximal end of the horn 32 to the electric cable 36. Note that the cover body 51 covering the multilayer transducer 41 has the bend preventer 52 in proximal end part, where the bend preventer 52 covers wiring lines 36a and 36b of the electric cable 36 electrically connected to two FPCs (flexible printed circuit) 47 and 48.

The multilayer transducer 41 has rectangular (quadrangular prism-shaped) insulating plates 42 and 43 of ceramics or the like disposed in front and behind, and is bonded on a forward side to a front mass 39, which is a rectangular (quadrangular prism-shaped) metal block body in this case, via the insulating plate 42 while being bonded on a rearward side to a back mass 44, which is a rectangular (quadrangular prism-shaped) metal block body, via the insulating plate 43, where the front mass 39 is formed integrally with the horn 32. Note that the front mass 39 and back mass 44 are formed of duralumin or a titanium alloy such as 64Ti (Ti-6Al-4V).

The multilayer transducer 41 is made of a non-lead monocrystalline material such as lithium niobate (LiNb03) having heat resistance and constructed by stacking plural, namely four in this case, monocrystalline piezoelectric chips 61, which are monocrystalline piezoelectric bodies. Positive-side bonding metal 62 and negative-side bonding metal 63 formed, for example, of AuSn eutectic solder, typical non-lead solder, or the like are interposed alternately among the insulating plate 42, multiple monocrystalline piezoelectric chips 61, and insulating plate 43, bonding together the plates and chips, where the positive-side bonding metal 62 serves as positive electrode layer and bonding layer while the negative-side bonding metal 63 serves as negative electrode layers and bonding layers. Note that while not serving as electrode layers, bonding metal layers formed, for example, of AuSn eutectic solder, typical non-lead solder, or the like are interposed between the front mass 39 and insulating plate 42 as well as between the insulating plate 43 and back mass 44 to bond together the front mass 39 and insulating plate 42 as well as to bond together the insulating plate 43 and back mass 44.

### (Production Method for Ultrasound Transducer)

Now, a production method for the ultrasound transducer 2 as a multilayer ultrasound vibration device will be described below.

A piezoelectric material which has a high Curie point and whose piezoelectric characteristics do not deteriorate even at a melting point of the bonding metal is used as a material for the ultrasound transducer 2. In this case, the ultrasound transducer 2 is created from a LiNb03 wafer 40 (see Fig. 6).

First, as shown in Figs. 7 and 8, on both surfaces of the LiNb03 wafer 40, V-shaped grooves 64 to become chamfered portions 66 described later are formed by dicing at corresponding locations in front and back surfaces of the wafer along dashed lines B in Fig. 6. Note that the grooves 64 in the LiNb03 wafer 40, may be formed by etching or patterning of the photoresist. In so doing, depth of the grooves 64 needs to be set to no more than half the thickness of the LiNb03 wafer 40 to avoid dicing of the LiNb03 wafer 40 and kept small enough to maintain sufficient mechanical strength so that the LiNb03 wafer 40 will not be broken in subsequent processes.

Next, a film of an underlying metal 65 is formed by vapor deposition, sputtering, plating or the like on both surfaces of the LiNb03 wafer 40 with the grooves 64 formed therein as shown in Fig. 9, where the underlying metal 65 is made, for example, of Ti/Ni/Au, Ti/Pt/Au, Cr/Ni/Au, or Cr/Ni/Pd/Au, which have good adhesion to and wettability by AuSn eutectic solder, non-lead solder, or the like, which is to become the positive-side bonding metal 62 and negative-side bonding metal 63. In this way, by forming a film of the underlying metal 65 after the grooves 64 are formed in the LiNb03 wafer 40, the film of the underlying metal 65 is formed in the grooves 64 as well.

Finally, using a dicing blade smaller in thickness than width of the grooves 64, those portions of the LiNb03 wafer 40 in which the grooves 64 are formed in advance are cut again along center portions of the grooves 64, thereby dicing the LiNb03 wafer 40 into plural monocrystalline piezoelectric chips 61 as shown in Fig. 10. In this way, as shown in Fig. 11, the monocrystalline piezoelectric chips 61 as monocrystalline piezoelectric elements are produced with side edges cut off, with chamfered portions 66 formed on an entire circumference, and with a film of the underlying metal 65 formed on two surfaces, i.e., front and back surfaces (top and bottom surfaces in Fig. 11), as well as on each of the chamfered portions 66.

Next, the ultrasound transducer 2 containing the multilayer transducer 41 is assembled, where the multilayer transducer 41 uses the monocrystalline piezoelectric chips 61, four in number in this case, resulting from the dicing. First, as in the case of the LiNb03 wafer 40, a film of the underlying metal 65 is formed on each of the following surfaces: one surface (rearward end face) of the front mass 39 formed integrally with the horn 32, both surfaces of the two insulating plates 42 and 43, and one surface (forward end face) of the back mass 44. As shown in Fig. 12, on the underlying metal 65 of the front mass 39 formed integrally with the horn 32, the two insulating plates 42 and 43, the back mass 44, and the plural monocrystalline piezoelectric chips 61, AuSn eutectic solder, non-lead solder, or the like is disposed by screen printing or in ribbon form.

Then, as shown in Fig. 13, the horn 32 having the front mass 39, the insulating plate 42, the plural monocrystalline piezoelectric chips 61, four in number here, the insulating plate 43, and the back mass 44 are stacked in this order starting from the distal end side. As the stacked structure is heated to or above melting temperature of AuSn eutectic solder, non-lead solder, or the like and then cooled slowly, the positive-side bonding metal 62 or negative-side bonding metal 63 is formed between each pair of adjacent ones of the insulating plate 42 on the distal end side, the plural monocrystalline piezoelectric chips 61, four in number here, and the insulating plate 43 on a proximal end side, forming bonding metal layers between the front mass 39 and insulating plate 42 as well as between the insulating plate 43 and back mass 44 and resulting in integration. Note that it is advisable to apply pressure to the ultrasound transducer 2 so as to compress the ultrasound transducer 2 in a longitudinal axial direction, as required, during heating.

Regarding thicknesses of the positive-side bonding metal 62 and negative-side bonding metal 63, since all the side edges on the two surfaces, i.e., front and back surfaces, of the monocrystalline piezoelectric chips 61 are cut off, forming respective chamfered portions 66, a thickness d2 or d3, in the stacking direction, between each pair of adjacent outer surfaces in a circumferential direction around a longitudinal direction of the ultrasound transducer 2 is made larger than an inward thickness d1, which is a spacing distance between opposing surfaces of each pair of adjacent ones of the monocrystalline piezoelectric chips 61, insulating plate 42, and insulating plate 43 along a stacking direction (d1 < d2; d1 < d3). That is, the positive-side bonding metal 62 and negative-side bonding metal 63 are shaped to be wider (thickness d2 or d3) along the stacking direction in outer surface portions on an outward side than an inward-side thickness d1 corresponding to a facing distance between each pair of adjacent ones of the monocrystalline piezoelectric chips 61 and insulating plates 42 and 43. Note that the respective wide outer surfaces of the positive-side bonding metal 62 and negative-side bonding metal 63 make up part of side faces of the multilayer transducer 41 and some of these planes make up electrically connecting planar portions 62a and 63a described later. Also, chamfered portions 66 are not formed on the insulating plates 42 and 43, and consequently the negative-side bonding metal 63 is configured such that the thickness d2 of the outer surface between the monocrystalline piezoelectric chips 61 along the stacking direction is larger than the thickness d3 of the outer surface between the insulating plate 42 or 43 and the monocrystalline piezoelectric chip 61 (d2>d3). Of course, chamfered portions 66 may be formed on the side of those faces of the insulating plates 42 and 43 which face the monocrystalline piezoelectric chips 61.

As shown in Figs. 14 and 15, the positive-side bonding metal 62 and negative-side bonding metal 63 formed in this way are electrically connected with electric contacts serving as conductive portions in the FPCs 47 and 48 via electric connection portions 49 formed using solder, electric conductive paste, or the like. That is, in order to electrically connect the positive-side bonding metal 62 and negative-side bonding metal 63 with the respective FPCs 47 and 48, the electrically connecting planar portions 62a and 63a which are to become the wide outer surfaces of the positive-side bonding metal 62 and negative-side bonding metal 63 make contact with the electric contacts of the FPCs 47 and 48 via the electric connection portions 49, and consequently the FPCs 47 and 48 are fixed to the ultrasound transducer 2 by being electrically connected to the positive-side bonding metal 62 and negative-side bonding metal 63.

In this way, an electrical connection is established between the positive-side bonding metal 62 and FPC 47 as well as between the negative-side bonding metal 63 and FPC 48. Then, the FPCs 47 and 48 are connected with the wiring lines 36a and 36b of the electric cable 36 (see Figs. 4 and 5) described above. Thus, a drive signal of the ultrasound transducer 2 is transmitted to the electrically connecting planar portions 62a making up part of a side face of the multilayer transducer 41 as well as to the positive-side bonding metal 62 via the wiring lines 36a and 36b of the electric cable 36, the FPCs 47 and 48, and the electric connection portions 49, applied to each of the monocrystalline piezoelectric chips 61, and fed back from the negative-side bonding metal 63 and electrically connecting planar portions 63a.

Note that surface portions in which the positive-side bonding metal 62, negative-side bonding metal 63, and electric connection portions 49 are exposed may be covered by insulating material such as resin to prevent unnecessary electrical connections which could become faulty or that the FPCs 47 and 48 may be fixed to the positive-side bonding metal 62 and negative-side bonding metal 63 with an adhesive in order to reinforce mechanical fixing of the FPCs 47 and 48. Furthermore, the FPCs 47 and 48 may be fixed to surfaces of each of the monocrystalline piezoelectric chips 61 with an adhesive.

In the production process described above, the front mass 39 formed integrally with the horn 32, the multiple insulating plates 42, the four monocrystalline piezoelectric chips 61, and the back mass 44 are stacked and integrated by the positive-side bonding metal 62 and negative-side bonding metal 63, which are to become bonding metal layers. Consequently, the entire ultrasound transducer 2 goes through ultrasound vibration when a drive signal is applied to the positive-side bonding metal 62 from the FPCs 47 and 48 provided on the side faces of the stacked structure via the electric connection portions 49 and fed back from the negative-side bonding metal 63.

The ultrasound transducer 2 adapted to function as a Langevin transducer and configured as described above allows the thickness d2 (d3) (width) of the positive-side bonding metal 62 and negative-side bonding metal 63 along the stacking direction to be increased in outer surface portions even if the thickness d1 of the positive-side bonding metal 62 and negative-side bonding metal 63 is reduced, where the positive-side bonding metal 62 and negative-side bonding metal 63 serve as metal bonding layers adapted to bond the insulating plates 42 and 43 or the plural monocrystalline piezoelectric chips 61 while the positive-side bonding metal 62 and negative-side bonding metal 63 provide electric connection with the FPCs 47 and 48. Thus, by increasing the thickness d2 (d3) of only outer circumferential part, while keeping down the thickness d1 of most of inner part (inward part) out of portions which bond the plural monocrystalline piezoelectric chips 61, it is possible to improve reliability of electrical connection while at the same time preventing degradation of vibration performance.

Thus, the ultrasound transducer 2 according to the present embodiment, which is a Langevin transducer serving as a multilayer ultrasound vibration device, is configured to be able to improve the reliability of electrical connection in that part which forms the electrical connection (electric connection portions 49) between wiring lines (FPCs 47 and 48) adapted to apply a drive signal and metallic bonding material (positive-side bonding metal 62 and negative-side bonding metal 63) while preventing degradation of vibration performance.

Note that although in the example described above, the front mass 39 formed integrally with the horn 32, the multiple insulating plates 42, the four monocrystalline piezoelectric chips 61, and the back mass 44 are rectangular block bodies, this is not restrictive and shapes of these components may be, for example, circular cylindrical shapes as shown in Fig. 17. Note that chamfered portions 66 are also formed on monocrystalline piezoelectric chips 61 in this case by cutting off outer circumferential edges on both surfaces.

### (Second Embodiment)

Next, a second embodiment of the present invention will be described below with reference to drawings. Note that the same components as those in the first embodiment are denoted by the same reference numerals as the corresponding components in the first embodiment, and thus detailed description thereof will be omitted.

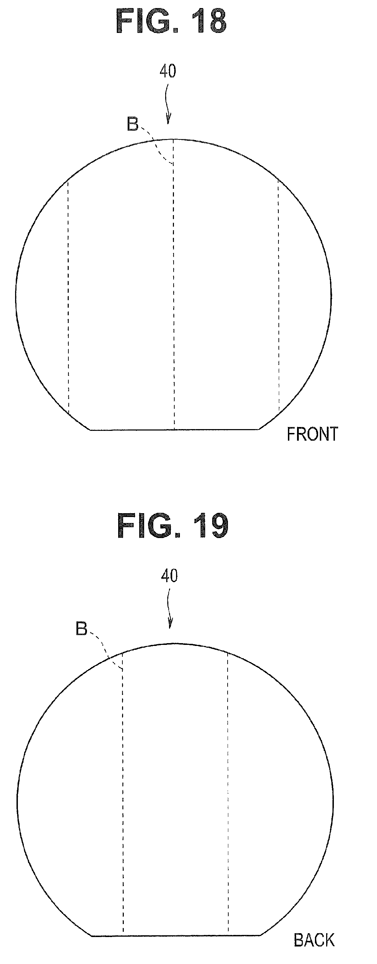

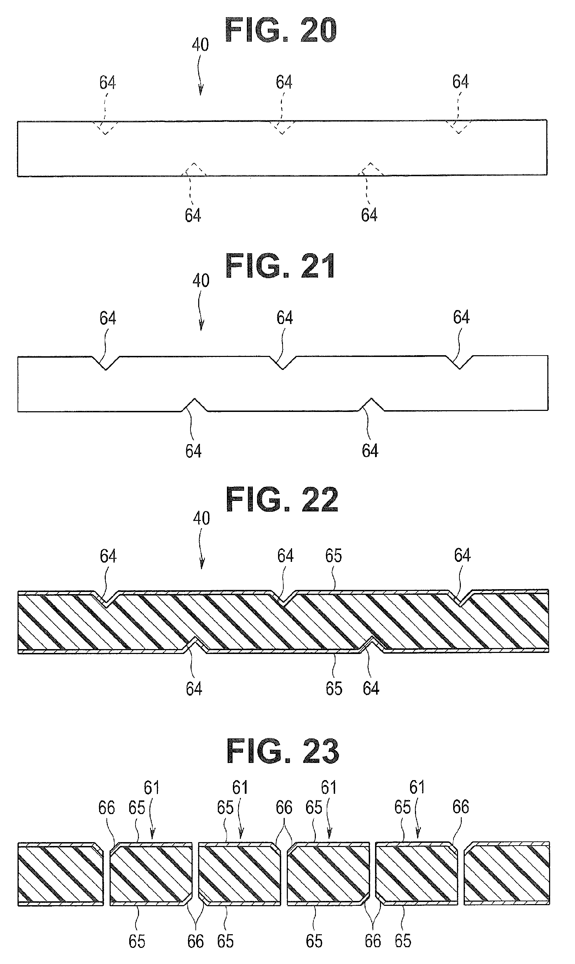

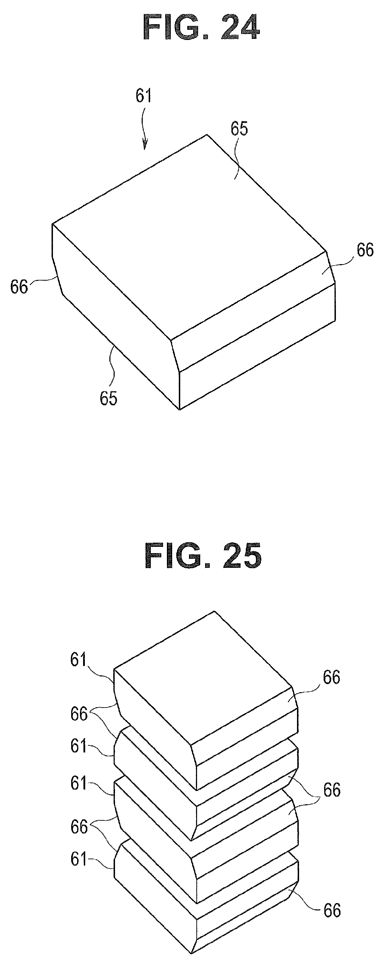

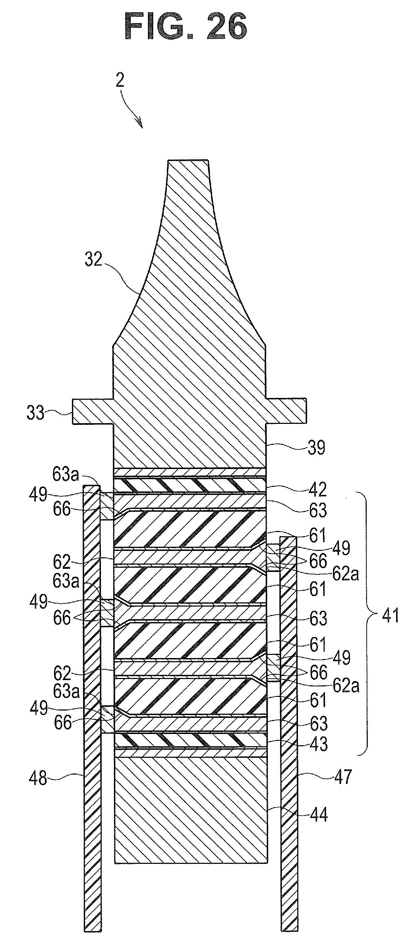

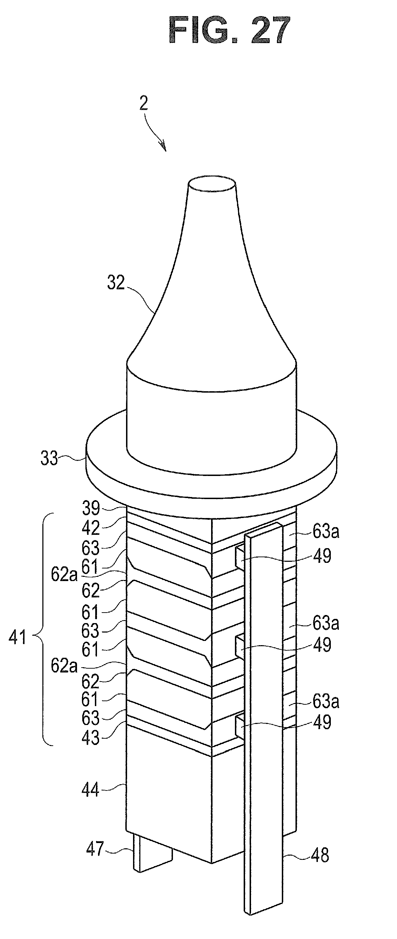

Fig. 18 is a plan view showing a surface of a LiNb03 wafer, Fig. 19 is a plan view showing a back surface of the LiNb03 wafer, Fig. 20 is a side view showing a configuration of the LiNb03 wafer, Fig. 21 is a side view showing the configuration of the LiNb03 wafer with grooves formed therein, Fig. 22 is a sectional view showing the configuration of the LiNb03 wafer with a film of underlying metal formed thereon, Fig. 23 is a sectional view showing a configuration of plural monocrystalline piezoelectric chips cut from the LiNb03 wafer, Fig. 24 is a perspective view showing the configuration of the monocrystalline piezoelectric chip, Fig. 25 is an exploded perspective view showing a configuration of four monocrystalline piezoelectric chips, Fig. 26 is a sectional view showing the configuration of the ultrasound transducer, and Fig. 27 is a perspective view showing the configuration of the ultrasound transducer.

Here, in the ultrasound transducer 2 as a multilayer ultrasound vibration device, V-shaped grooves 64 are formed during production by dicing the LiNb03 wafer 40 at different locations in front and back surfaces of the wafer along dashed lines B in Figs. 18 and 19. That is, on the LiNb03 wafer 40, as shown in Figs. 20 and 21, the V-shaped grooves 64 in the front surface and back surface of the wafer are formed in a staggered manner so as to be placed at locations different between the front and back surfaces in a direction orthogonal to a thickness direction of the wafer.

In this case again, as shown in Fig. 22, a film of the underlying metal 65 is formed on both surfaces of the LiNb03 wafer 40 with the grooves 64 formed therein. Next, the LiNb03 wafer 40 is cut again along center lines of the grooves 64 as well as at predetermined locations in a direction orthogonal to the grooves 64, and thereby diced into plural monocrystalline piezoelectric chips 61 as shown in Fig. 23. In this way, as shown in Fig. 24, the monocrystalline piezoelectric chips 61 as monocrystalline piezoelectric elements are produced with side edges in a diagonal direction on one of the front and back surfaces cut off, forming two chamfered portions 66, and with a film of the underlying metal 65 formed again on two surfaces, i.e., front and back surfaces (top and bottom surfaces in Fig. 24), as well as on the two chamfered portions 66.

As shown in Fig. 25, the four monocrystalline piezoelectric chips 61 produced in this way are stacked via AuSn eutectic solder, non-lead solder, or the like (not shown) in such a way that two faces located opposite each other with the respective chamfered portions 66 formed thereon are put together and that the chamfered portions 66 will face each other. That is, the monocrystalline piezoelectric chips are stacked such that the chamfered portions 66 of the two monocrystalline piezoelectric chips 61 placed on opposite ends and the chamfered portions 66 of the two monocrystalline piezoelectric chips 61 placed on an inner side, facing the two outer chips, will face each other in close vicinity and furthermore such that the chamfered portions 66 formed on opposing faces of the two monocrystalline piezoelectric chips 61 placed on the inner side will face each other in close vicinity.

Thus, as with the first embodiment, the four monocrystalline piezoelectric chips 61 resulting from dicing and stacked have a film of underlying metal 65 formed thereon, get stacked together with the horn 32 having the front mass 39 with AuSn eutectic solder, non-lead solder, or the like provided thereon, the insulating plates 42, the insulating plate 43, and the back mass 44, and undergo a heating and cooling process, and consequently the ultrasound transducer 2 is produced with the bonding metal layers, positive-side bonding metal 62, and negative-side bonding metal 63 formed thereon (see Fig. 26).

In this way, in the ultrasound transducer 2 according to the present embodiment, since each chamfered portion 66 is formed by cutting off a side edge on one side of each of two surfaces, i.e., front and back surfaces, of the monocrystalline piezoelectric chips 61, the positive-side bonding metal 62 and negative-side bonding metal 63 are made wider (thicker) on two opposite side faces of the multilayer transducer 41 than on the other two side faces. Then, some planes of the two wider side faces make up electrically connecting planar portions 62a and 63a, which are electrically connected with the FPCs 47 and 48 via the electric connection portions 49 as shown in Fig. 26. Again, the FPCs 47 and 48 are connected with the wiring lines 36a and 36b of the electric cable 36 (see Figs. 4 and 5) described above.

The ultrasound transducer 2 according to the present embodiment adapted to function as a Langevin transducer and configured as described above can improve the reliability of electrical connection in that part which forms the electrical connection (electric connection portions 49) between wiring lines (FPCs 47 and 48) adapted to apply a drive signal and metallic bonding material (positive-side bonding metal 62 and negative-side bonding metal 63) as described in the first embodiment, offer the effect of preventing degradation of vibration performance, reduce processing locations of dicing which involves forming the grooves 64 in the LiNb03 wafer 40, cutting the LiNb03 wafer 40 into pieces, and forming the chamfered portions 66 on the monocrystalline piezoelectric chips 61, and thereby slash production cost of the monocrystalline piezoelectric chips 61.

Furthermore, with the ultrasound transducer 2 after assembly, since no chamfered portion 66 is formed on a portion of the monocrystalline piezoelectric chip 61 on each side face which is not electrically connected, the positive-side bonding metal 62 and negative-side bonding metal 63 are reduced in width (thickness) and an exposed portion remains small, offering the advantage of being able to make unnecessary electrical connections less prone to occur when the electric contacts of the FPCs 47 and 48 are connected with the electric connection portions 49 made of solder, electric conductive paste, or the like.

### (First Modification)

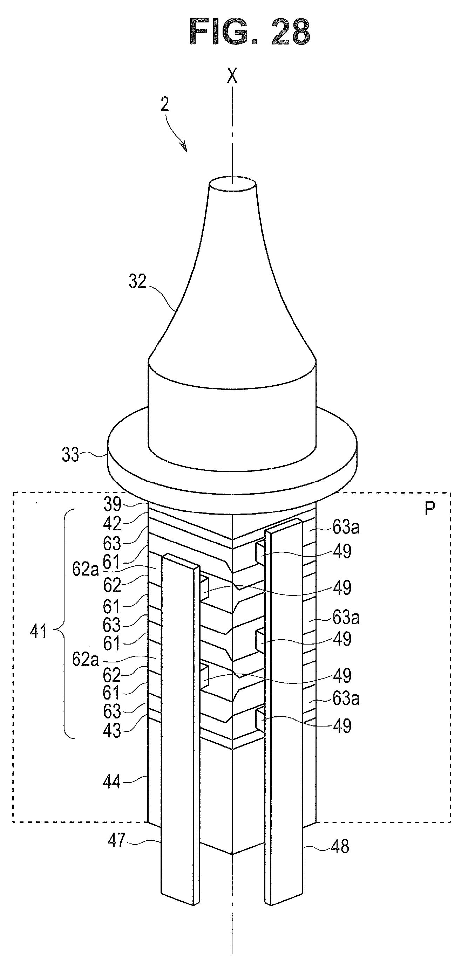

Fig. 28 is a perspective view showing a configuration of an ultrasound transducer.

As shown in Fig. 28, the ultrasound transducer 2 here is configured such that the electric contacts of the FPCs 47 and 48 are connected to the electrically connecting planar portions 62a and 63a of the positive-side bonding metal 62 and negative-side bonding metal 63 on two side faces via the electric connection portions 49 formed using solder, electric conductive paste, or the like, where the two side faces are demarcated by a central axis X in a longitudinal direction and a plane P passing through opposite edges of the multilayer transducer 41. In other words, the FPCs 47 and 48 are electrically connected to the electrically connecting planar portions 62a and 63a of the positive-side bonding metal 62 and negative-side bonding metal 63 on two adjacent side faces of the multilayer transducer 41 via the electric connection portions 49. That is, four monocrystalline piezoelectric chips 61 are stacked such that the chamfered portions 66 are aligned, forming the electrically connecting planar portions 62a and 63a on the adjacent two side faces of the multilayer transducer 41.

With such a configuration, in a process of fixing the FPCs 47 and 48 to the side faces of the multilayer transducer 41 to electrically connect the FPCs 47 and 48 to the positive-side bonding metal 62 and negative-side bonding metal 63 via the electric connection portions 49, since no FPC 47 or 48 exists on two side faces on opposite sides of the two side faces of the multilayer transducer 41 on which the FPCs 47 and 48 are mounted unlike each of the embodiments described above, the ultrasound transducer 2 can be fixed easily, simplifying a step of mounting the FPCs 47 and 48 by electrically connecting with the electrically connecting planar portions 62a and 63a.

### (Second Modification)

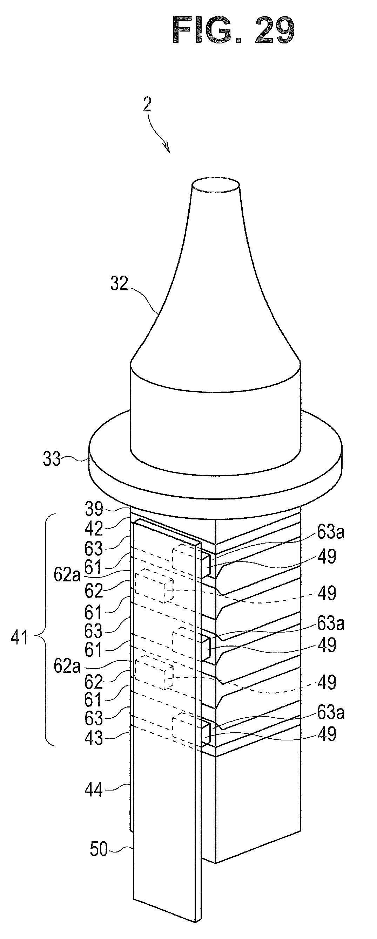

Fig. 29 is a perspective view showing a configuration of an ultrasound transducer.

As shown in Fig. 29, in the ultrasound transducer 2 here, in order to apply positive and negative voltages necessary for driving to the monocrystalline piezoelectric chips 61, on one side face of the multilayer transducer 41, one FPC 50 is electrically connected to the electrically connecting planar portions 62a and 63a of the positive-side bonding metal 62 and negative-side bonding metal 63 via the electric connection portions 49. Note that the ultrasound transducer 2 may be configured such that the two FPCs 47 and 48 are fixed to one side face of the multilayer transducer 41. Again, four monocrystalline piezoelectric chips 61 are stacked such that the chamfered portions 66 are aligned, forming the electrically connecting planar portions 62a and 63a on one side face of the multilayer transducers 41.

Although in the configuration described above as an example, multilayer transducer 41 of the ultrasound transducer 2 is shaped as rectangular parallelepipeds, this is not restrictive and the multilayer transducer 41 may have any shape such as a prism or a cylinder as long as ultrasound vibrations can be excited.

Furthermore, the shapes of the horn 32, outward flange 33 and, front mass 39 do not have to be formed in advance, and may be formed by cutting work after the insulating plates 42 and 43, monocrystalline piezoelectric chips 61, and back mass 44 are integrated into a metal block body.

As described above, in the ultrasound transducer 2 according to the present invention, which is a Langevin transducer, the chamfered portions 66 are provided on end portions of the monocrystalline piezoelectric chips 61 to electrically connect the FPCs 47, 48, and 50 serving as wiring lines for use to apply a drive signal with the positive-side bonding metal 62 and negative-side bonding metal 63 used as metallic bonding material, the wide electrically connecting planar portions 62a and 63a are formed on one or two side faces of the multilayer transducers 41, and the electric connection portions 49 for use to connect to the FPCs 47, 48, and 50 are formed on the electrically connecting planar portions 62a and 63a. This makes it possible to increase a bonding area between the electrically connecting planar portions 62a and 63a and the electric connection portions 49 and thereby improve the reliability of the electrical connections.

Thus, according to the present invention, from a viewpoint of the vibration performance of the ultrasound transducer 2, preferably the positive-side bonding metal 62 and negative-side bonding metal 63 used as the bonding metal formed among the plural monocrystalline piezoelectric chips 61 have small thickness and preferably the electrically connecting planar portions 62a and 63a of the positive-side bonding metal 62 and negative-side bonding metal 63 are thick (large in area) only in portions where electrical connections are made. Therefore, degradation in the vibration performance of the ultrasound transducer 2 can be prevented by keeping most of inner part of the positive-side bonding metal 62 and negative-side bonding metal 63 thin. At the same time, the reliability of the electrical connection with the FPCs 47, 48, and 50 can be improved by thickening only outer circumferential portions of the positive-side bonding metal 62 and negative-side bonding metal 63 and thereby increasing the width of the electrically connecting planar portions 62a and 63a.

The invention described in the above embodiments is not limited to the embodiments and modifications, and may be modified in various forms in the implementation stage without departing from the spirit or scope of the invention. Furthermore, the embodiments described above include inventions at various stages, and various inventions can be extracted through appropriate combinations of the disclosed components.

For example, even if some of the components are removed from any of the embodiments, the resulting configuration can be extracted as an invention as long as the configuration can solve the problems described above and provide the advantages described above.

The present application is based upon and claims the benefit of priority from Japanese Patent Application No. 2013-055504, filed in Japan on March 18, 2013, the entire contents of which are incorporated in the specification, claims, and drawings of Japanese Patent Application No. 2013-055504 by reference.

## Claims

1. A multilayer ultrasound vibration device, comprising a plurality of piezoelectric bodies and a plurality of electrode layers stacked together, wherein: the plurality of piezoelectric bodies are formed of a plurality of monocrystalline piezoelectric bodies; and width of outer surfaces of the plurality of electrode layers in a stacking direction is made larger than a spacing distance of the stacked plurality of monocrystalline piezoelectric bodies in the stacking direction.

2. The multilayer ultrasound vibration device according to claim 1, wherein only the outer surfaces of the plurality of electrode layers which are connected with a wiring line adapted to supply a drive signal have the width in the stacking direction made larger than the spacing distance of the plurality of monocrystalline piezoelectric bodies in the stacking direction.

3. The multilayer ultrasound vibration device according to claim 1, wherein chamfered portions are formed on side edges of the plurality of monocrystalline piezoelectric bodies such that an outward-side thickness is larger than an inward-side thickness of the plurality of electrode layers and the width of the plurality of electrode layers in the stacking direction is made larger on a side of the outer surfaces of the plurality of electrode layers.

4. The multilayer ultrasound vibration device according to either claim 2 or 3, wherein: a prismatic stacked structure is formed by the plurality of monocrystalline piezoelectric bodies and the plurality of electrode layers; and the chamfered portions are formed on at least one of side edges of the plurality of monocrystalline piezoelectric bodies.

5. The multilayer ultrasound vibration device according to claim 4, wherein the outer surfaces of the plurality of electrode layers connected with the wiring line are formed on adjacent two side faces of the prismatic stacked structure.

6. The multilayer ultrasound vibration device according to any one of claims 1 to 5, wherein the plurality of electrode layers are metal members, which are of a bonding metal adapted to fuse together and thereby integrate the plurality of monocrystalline piezoelectric bodies to be stacked.

7. The multilayer ultrasound vibration device according to any one of claims 3 to 6, wherein the monocrystalline piezoelectric bodies are produced by forming V-shaped grooved portions in front and back surfaces of a monocrystalline piezoelectric wafer by a dicing process, and dicing the monocrystalline piezoelectric wafer into a plurality of pieces along centers of the grooved portions by the dicing process while making the chamfered portions out of the cut grooved portions.

8. The multilayer ultrasound vibration device according to claim 6, wherein the monocrystalline piezoelectric bodies are produced by forming V-shaped grooved portions in front and back surfaces of a monocrystalline piezoelectric wafer by a dicing process, forming a film of underlying metal on the front and back surfaces of the monocrystalline piezoelectric wafer to enhance bonding strength with the bonding metal to be melted, and dicing the monocrystalline piezoelectric wafer into a plurality of pieces along centers of the grooved portions by the dicing process while making the chamfered portions out of the cut grooved portions.

9. A production method for the multilayer ultrasound vibration device according to any one of claims 1 to 6, comprising: forming V-shaped grooved portions in front and back surfaces of a monocrystalline piezoelectric wafer by a dicing process; dicing the monocrystalline piezoelectric wafer into the plurality of monocrystalline piezoelectric bodies along centers of the grooved portions by the dicing process while making the chamfered portions out of the cut grooved portions; and placing a bonding metal on the plurality of monocrystalline piezoelectric bodies, applying a heating and cooling process thereto, thereby bonding the plurality of monocrystalline piezoelectric bodies into one body, and forming the plurality of electrode layers from the bonding metal.

10. The production method for the multilayer ultrasound vibration device according to claim 9, further comprising forming a film of underlying metal on the front and back surfaces of the monocrystalline piezoelectric wafer to enhance bonding strength with the bonding metal to be melted, after forming the V-shaped grooved portions in the front and back surfaces of the monocrystalline piezoelectric wafer by the dicing process.

11. An ultrasound medical apparatus comprising: the multilayer ultrasound vibration device according to any one of claims 1 to 8; and a probe distal end portion used to treat living tissue with ultrasound vibrations generated by the multilayer ultrasound vibration device and transmitted to the probe distal end portion.
